# EUROPÄISCHE PATENTANMELDUNG

(11) **EP 0 839 917 A1**
(43) Veröffentlichungstag der Anmeldung: **06.05.1998**
(21) Anmeldenummer: 97118318.1
(22) Anmeldetag: 22.10.1997
(51) Int. Cl.: C12Q 1/70, C12Q 1/68

(54) **Primer und Sonden zum Nachweis von HIV**

(30) Priorität: 24.10.1996 DE 19644248
(71) Anmelder: BOEHRINGER MANNHEIM GMBH, 68298 Mannheim (DE)
(72) Erfinder: Kasper, Pia, Dr., 82436 Obereglfing (DE)

(57) **Zusammenfassung**

Mit Oligonukleotiden aus dem 3'-Endbereich des gag-Gens von HIV-1 sind bedeutend mehr Subtypen von HIV nachweisbar als mit den bisher bekannten. Dies erhöht die Sicherheit der HIV-1-Bestimmungen

## Beschreibung

Gegenstand der Erfindung sind Oligonukleotide, insbesondere Primer und Sonden, zum Nachweis von HIV. Ebenfalls Gegenstand der Erfindung sind Verfahren zum Nachweis von HIV mit Hilfe dieser Primer und Sonden.

Der Nachweis des Humanen Immundefizienzvirus (HIV) ist eine der großen Herausforderungen der analytischen Diagnostik. Es existieren hierfür bereits zahlreiche Verfahren, die entweder auf immunologischen Nachweisen von HIV-Antigenen oder durch HIV-induzierten Antikörpern, HIV-eigenen Enzymen, z. B. der HIV-eigenen reversen Transkriptase, und HIV-spezifischen Nukleinsäuren beruhen. Da der Erreger und somit auch die mit ihm assoziierten Nukleinsäuren nur in sehr geringen Konzentrationen in menschlichen Körperflüssigkeiten vorkommen, ist die Sensitivität eines Nachweisverfahrens ein entscheidender Faktor für die Verwendbarkeit dieses Nachweisverfahrens. Zum direkten Nachweis von HIV ist die Polymerase-Kettenreaktion (PCR), wie beispielsweise beschrieben in EP-B-0 200 362 und EP-B-0 201 184, die derzeit sensitivste Methode. Mit ihrer Hilfe können wichtige Hinweise auf Krankheitsverlauf und Therapiemonitoring aus dem Blut Infizierter gewonnen werden. Nachweisverfahren, die nicht auf einer Amplifikation von Teilen der HIV-Nukleinsäuren beruhen, oder bei denen eine einzige Hybridisierung einer Sonde, mit der HIV-Nukleinsäure detektiert wird, sind derzeit nicht so empfindlich wie die PCR.

Seit der Entdeckung des HIV-1 wurde festgestellt, daß die Nukleinsäuresequenzen von HIV-1 unterschiedlicher Provenienz sich unterscheiden. Die unterschiedlichen Typen von HIV-1 werden üblicherweise als Subtypen bezeichnet. Derzeit sind mindestens neun Subtypen bekannt, die als Subtypen A bis H und O bezeichnet werden (z. B. Human Retroviruses und AIDS, Los Alamos Natl. Laboratory, Los Alamos, New Mexico, 1994; ed. G. Myers et al., I-A-1). Die bisher beschriebenen Verfahren sind nicht in der Lage, fünf oder mehr dieser Subtypen zu detektieren, insbesondere nicht die Subtypen A und G zusammen mit anderen Subtypen.

Aufgabe der vorliegenden Erfindung war es daher, Reagenzien und Verfahren bereitzustellen, mit denen mehr Subtypen als bisher detektiert werden können.

Gegenstand der Erfindung ist daher ein HIV-1-spezifisches Oligonukleotid, dadurch gekennzeichnet, daß seine Sequenz ausgewählt ist aus dem Bereich der Nukleotidpositionen 900 bis zum 3'-Ende des gag-Gens von HIV-1.

Unter einem Oligonukleotid wird ein Molekül verstanden, welches an einem im wesentlichen aus identischen Monomereinheiten aufgebauten Rückgrat in definierten Abständen eine Aufeinanderfolge von Basen aufweist. Die Basen sind so an dem Rückgrat angeordnet, daß sie mit einer Nukleinsäure mit einer Sequenz von zu den Basen des Oligonukleotids komplementären Basen eine Bindung eingehen können. Die gebräuchlichsten Oligonukleotide enthalten ein Rückgrat von Zuckerphosphateinheiten. Man unterscheidet hier zwischen Oligodesoxyribonukleotiden, welche in 2'-Einheit keine Hydroxylgruppe aufweisen und Oligoribonukleotiden, welche in dieser Position eine Hydroxylgruppe aufweisen. Zu den Oligoribonukleotiden zählt man jedoch auch Verbindungen, bei denen der Wasserstoff der Hydroxylgruppe durch organische Reste, z. B. einer Allylgruppe, ersetzt ist. Solche Verbindungen sind dem Fachmann schon seit längerem bekannt. In jüngerer Zeit wurden auch Moleküle beschrieben, bei denen das Zuckerphosphatrückgrat von normalen Oligonukleotiden durch ein Peptidrückgrat ersetzt ist. Eine besonders bevorzugte Gruppe von solchen Verbindungen ist in WO 92/20702 beschrieben; sie werden PNA genannt. Diesen ist mit Oligonukleotiden gemeinsam, daß sie am Rückgrat Basen aufweisen, die zu Wasserstoffbrückenbindungen mit hierzu komplementären Basen in der Lage sind. Zu den Basen gehören einerseits die natürlichen Basen A, G, C, T und U, andererseits jedoch auch künstliche Basen, wie Deaza G.

Die erfindungsgemäßen Oligonukleotide sind bevorzugt zwischen 10 und 100 Basen lang. Hiervon haben mindestens 10 bis 30 Basen eine Homologie oder Komplementarität aufeinanderfolgender Basen von 80 %, bevorzugt 90 %, besonders bevorzugt 100 % zu dem oben genannten Nukleotidpositionenbereich. Die übrigen Basen (z. B. an einem oder beiden Enden der Sequenz) können, sofern vorhanden, eine Sequenz aufweisen, die weder zu HIV-1 noch zu anderen in einer üblicherweise auf HIV-1 zu untersuchenden Probe enthaltenden Nukleinsäure so große Ähnlichkeiten hat, daß eine Hybridisierung stattfinden könnte. Besonders bevorzugt ist der HIV-1-spezifische Bereich der erfindungsgemäßen Oligonukleotide zwischen 13 und 25 Nukleotiden lang.

Als zu 100 % komplementär zu einer zweiten Nukleotidsequenz wird im Sinne der Erfindung eine erste Sequenz bezeichnet, wenn die darin enthaltenen aufeinanderfolgenden Basen der Watson-Crick-Regel zu aufeinanderfolgenden Basen der zweiten Nukleotidsequenz folgen. Es sind hierbei jedoch solche Austausche erlaubt, bei denen solche Basen, insbesondere unnatürliche Basen, anstelle der natürlichen Basen eingeführt sind, die sich jedoch in ihrer spezifischen Wechselwirkung mit der zu der ausgestauschten Base komplementären Base nicht wesentlich unterscheiden. Als zu einer zweiten Sequenz homolog wird im Sinne der Erfindung eine aufeinanderfolgende erste Sequenz von Basen verstanden, die zu einer dritten Nukleinsäure mit aufeinanderfolgenden Basen genauso komplementär ist wie die zweite Nukleotidsequenz von aufeinanderfolgenden Basen gleicher Länge.

Unter Nukleinsäuren von HIV-1 wird genomische RNA von Viren verstanden, die den HIV-Viren zugerechnet werden. Derzeit unterscheidet man bei HIV-1 zwischen den Subtypen A bis H und O. Als Komplement dieser Nukleinsäuren wird die hierzu komplementäre Sequenz verstanden.

Unter einem Primer wird ein Oligonukleotid verstanden, welches durch eine DNA-Polymerase unter Verwendung von Monodesoxyribonukleosidtriphosphaten und einer als Matrize dienenden Nukleinsäure verlängert werden kann. Bevorzugt weist dieser Primer an einem Ende, welches im mit der Matrize hybridisierten Zustand dem 5'-Ende der Matrizennukleinsäure zugewandt ist, eine 3'-Hydroxylgruppe auf.

Unter einem Set von Primern wird eine Kombination oder ein Gemisch von mindestens zwei Arten von Primern verstanden, von denen der erste Primer mit Hilfe der Matrizennukleinsäure derart unter Bildung eines Verlängerungsproduktes verlängert werden kann, daß der zweite Primer mit diesem Verlängerungsprodukt in einem in 3'-Richtung des verlängerbaren Ende des ersten Primers liegenden Bereich des Verlängerungsproduktes hybridisieren kann und daß das verlängerbare Ende des zweiten Primers in 5'-Richtung des Verlängerungsproduktes des ersten Primers zeigt. Die Auswahl von Primern, welche zur Durchführung der Polymerasekettenreaktion (PCR) geeignet sind und die dieser Definition entsprechen, ist in EP-B-0 201 184 beschrieben.

Unter einer Amplifikation von Nukleinsäuren wird die Vermehrung von Nukleinsäuren oder Teilbereichen dieser Nukleinsäuren verstanden. So werden beispielsweise durch geeignete Auswahl der Primersequenzen mit Hilfe der PCR bevorzugt Teile von Nukleinsäuren mit einer Länge von zwischen 100 und 300 Basen amplifiziert.

Der Ausdruck "Amplifikation" soll auch die Transkription von RNA in DNA einschließen.

Unter dem gag-Gen von HIV-1 wird das Gen verstanden, welches z. B. für p24 (Kapsidprotein), p17 (Matrixprotein) und p6 codiert. Innerhalb dieses Gens finden sich sowohl konservierte als auch relativ variable Sequenzregionen. Ein Kern der vorliegenden Erfindung ist die Verwendung von Sequenzen, welche zu einer aufeinanderfolgenden Sequenz von Basen innerhalb des Bereiches der Nukleotidpositionen 900 bis zum 3'-Ende dieses Gens entweder komplementär oder homolog sind. Besonders bevorzugt ist der Bereich der Nukleotidposition 900 bis zum 3'-Ende des gag-Gens von HIV-1, Subtyp B, Stamm HIV-RF (SEQ.ID.NO. 1, Human Retroviruses and AIDS 1994, I- A-1, Los Alamos National Laboratory, Los Alamos, New Mexico 87545, USA, Editor G. Myers et al.).

Unter HIV-1-Spezifität wird die Eigenschaft verstanden, daß die betreffende Nukleotidsequenz außer in HIV-1 nicht in Nukleinsäuren vorkommt, die in üblicherweise auf die Anwesenheit von HIV-1 getesteten Proben enthalten sind. Besonders bevorzugt ist die betreffende Sequenz völlig einzigartig, d. h. kommt auch in anderen Proben nicht vor. Unter einer spezifischen Amplifikation oder einem spezifischen Nachweis wird ein Verfahren verstanden, mit dem nur HIV-1, nicht jedoch andere Organismen oder Nukleinsäuren, die in der Probe enthalten sein könnten, amplifiziert bzw. nachgewiesen werden. HIV-1-Spezifität einer Sequenz der vorliegenden Erfindung heißt jedoch auch, daß die Sequenz so unspezifisch ist, daß mindestens 5 Subtypen von HIV-1 erkannt, amplifiziert bzw. nachgewiesen werden können.

Erfindungsgemäße Oligonukleotide können in dem genannten Bereich von Positionen mit einem speziellen, bevorzugten Verfahren identifiziert werden. Grundlage dieses Verfahrens ist die betreffende Sequenz eines Stammes eines Subtyps von HIV-1, bevorzugt des Subtyps B, besonders bevorzugt des Stammes RF. Aus dieser Sequenz werden, bevorzugt mittels eines Computerprogrammes, Teilsequenzen einer Länge von 20 bis 30 Basen mit einem GC-Gehalt zwischen 50 und 60 %, keiner Selbst-Komplementarität am 3'-Ende, die darüber hinaus keinen CG-run am 3'-Ende haben und die keine Palindrome beinhalten, ausgesucht. Die zu dieser Sequenz des Stammes gehörige Konsensussequenz des Subtyps wird, z. B. anhand des oben genannten Buches Human Retroviruses and AIDS, 1994, ermittelt. Die Konsensussequenz ist die Sequenz, die die in allen Stämmen am meisten vorkommende Base an der bestimmten Position enthält. Es wurden nur noch solche Konsensussequenzen weiterbearbeitet, die maximal 2 Mismatche zu den Konsensussequenzen der gleichen Positionen der übrigen Subtypen, bevorzugt der Subtypen A und C bis H, aufweisen. Sofern diese Sequenzen an ihrem 3'-Ende Mismatche zu einem der Stämme aufweisen, werden diese Sequenzen um diese Nukleotide (1 bis 2 Nukleotide vom Ende her) gekürzt.

Für die Auswahl von Primersequenzen werden die so gefundenen Oligonukletoidsequenzen zu Sets von je 2 Primern unterschiedlicher Sequenz zusammengeführt, dergestalt, daß die 5'-Enden der Primer zwischen 150 und 400 Basen voneinander positioniert sind.

Besonders bevorzugte Primersets sind solche, bei denen innerhalb dieses Bereiches eine weitere HIV-1-spezifische Sequenz liegt. Diese Sequenz kann besonders bevorzugt als Sonde zum Nachweis von Amplifikaten genutzt werden.

Nach diesem Verfahren wurden als besonders bevorzugte Oligonukleotide die folgenden Sequenzen gefunden:
GAG1037: TGATGACAGCATGTCAGGGAGTGG SEQ.ID.NO. 2
GAG1228: TCCACATTTCCAACACCCCTTTTT SEQ.ID.NO. 3
GAG1177: TTCAATTGTGGCAAAGAAGGG SEQ.ID.NO. 4
GAG1075: AAAGCAAGAATTTTGGCTGAAG SEQ.ID.NO. 5

Als besonders bevorzugt hat sich die Kombination GAG1037/1228 als Primerpaar erwiesen. Von den Sondenoligonukleotiden hat sich GAG1177 als besonders bevorzugt erwiesen.

Die erfindungsgemäßen Oligonukleotide können nach bekannten Verfahren hergestellt werden, z. B. nach der Festphasensynthese mit Phosphoramiditen. Hierfür gibt es geeignete Syntheseautomaten.

Die erfindungsgemäßen Oligonukleotide können sowohl als Primer als auch als Sonden verwendet werden. Bei Verwendung als Sonden ist es bevorzugt, daß das Oligonukleotid neben der HIV-spezifischen Sequenz eine nicht HIV-spezifische Markierung enthält. Hier kann es sich um jede beliebige unterscheidende Markierung handeln, z. B. eine Nukleinsäuresequenz, die nicht HIV-spezifisch ist, ein nachweisbares Molekül, z. B ein fluoreszierender Rest (der nach bekannten Verfahren mittels Fluoreszeinisothiocyanat einführbar ist), oder Digoxigenin, wie in EP-A-0 324 474 beschrieben, oder ein immobilisierbares Molekül, wie z. B. Biotin (über welches das Oligonukleotid beispielsweise an eine Streptavidinbeschichtete Oberfläche gebunden werden kann), sein.

Gegenstand der Erfindung ist daher auch ein Set von Primern zur Amplifikation von HIV-Nukleinsäuren, dadurch gekennzeichnet, daß es mindestens 2 unterschiedliche Oligonukleotide gemäß der obigen Definition enthält. Diese Sets von Primern können bevorzugt in Amplifikationsverfahren mit der Polymerasekettenreaktion oder der NASBA gemäß EP-A-0 329 822 eingesetzt werden. Die Grundzüge dieser Verfahren sind dem Fachmann bekannt.

Ebenfalls Gegenstand der Erfindung ist ein Verfahren zur spezifischen Amplifikation von HIV-1-Nukleinsäuren mit Hilfe eines Sets von Primern, mit dem Nukleinsäuren von mindestens 5 Subtypen von HIV-1 erkannt und amplifiziert werden. Besonders bevorzugt ist Subtyp B einer dieser erkannten Subtypen, besonders bevorzugt in Kombination mit einem oder mehreren der Subtypen A, C, D, E, F und G.

Ebenfalls Gegenstand der Erfindung ist ein Verfahren zum Nachweis von HIV-1 unter Amplifikation von HIV-1-Nukleinsäuresequenzen und Nachweis der Amplifikate, wobei die Amplifikation mit einem Primerset der oben beschriebenen Art vorgenommen wird. Besonders bevorzugt sind Nachweisverfahren, bei denen die gebildeten Amplifikate mit Hilfe einer Nukleinsäuresonde vorgenommen wird, die der obigen Definition der Oligonukleotide genügen und welche innerhalb der Primerhybridisierungsstellen im Amplifikat binden.

Die Erfindung hat den Vorteil, daß mit ihr mehr Subtypen von HIV-1 als bisher nachgewiesen werden können.

Die vorliegende Erfindung wird durch die folgenden Beispiele naher erläutert:

### Beispiel 1

### A. Probenvorbereitung

Die verwendeten Blutproben wurden zur Isolierung der RNA gemäß QIAamp HCV Kit (Qiagen Cat. No. 29504) vorbehandelt.

### B. Reverse Transkription

10 µl der erhaltenen Lösung wurden mit 10 µl RT-Mix folgender Zusammensetzung in PCR-Tubes in einem Perkin-Elmer Thermocycler 9600 behandelt (30 min bei 42 °C, 5 min bei 94 °C, abkühlen und halten bei 4 °C):

| Reagenz | Stock-Lösung | Volumen | Endkonzentration |
|---|---|---|---|
| 5 x RT-Puffer | 5 x | 4 µl | 1 x |
| dNTP-Mix | 10 mM | 0,5 µl | 250 µM |
| Hexamere | 20 µM | 1 µl | 1 µM |
| DEPC-H₂O | | 3,5 µl | |
| RNasin | 2 U/µl | 0,5 µl | 1 U |
| M.MuLV-RT | 20 U/µl | 0,5 µl | 10 U |

Dabei bedeuten:
- dNTP-Mix:: PCR-Nucleotide-Mix (Boehringer Mannheim GmbH, Cat. No. 1581295)
- RNasin:: RNase-Inhibitor (Boehringer Mannheim GmbH, Cat. No. 1277081))
- M.MuLV-RT, RT-Puffer:: Reverse Transkriptase (Boehringer Mannheim GmbH, Cat. No. 1062603)
- DEPC:: Aqua bidest., steril
- Hexamere:: Gemisch von Hexadesoxyribonukleotiden aller möglichen Sequenzen (Random Hexamers, Boehringer Mannheim GmbH, Cat. No. 1277081)

### C. PCR

20 µl der Mischung aus der RT wurden mit 80 µl PCR-Mix versetzt:

| Reagenz | Stock-Lösung | Volumen | Endkonzentration |
|---|---|---|---|
| 10 x PCR | 10 x | 10 µl | 1 x |
| DIG-dNTPs | | 10 µl | |
| 5'-Primer | 5 µM | 4 µl | 200 nM 20pmol/PCR |
| 3'-Primer | 5 µM | 4 µl | 200 nM 20 pmol/PCR |
| H₂O | | 51 µl | |
| Taq DNA Pol | 5 U/µl | 1 µl | 5 U |
| RT-Mischung | | 20 µl | |
| Endvolumen | | 100 µl | |

### Dabei bedeuten:

- 10 x PCR, DIG-dNTPs:: aus PCR Dig Labelling Mix (Cat. No. 1585550, Boehringer Mannheim GmbH
- 5'-Primer:: z. B. SEQ.ID-NO. 2
- 3'-Primer:: z. B. SEQ.ID.NO. 3
- Taq DNA-Polymerase:: (Boehringer Mannheim GmbH, Cat. No. 1146165)

Das Gemisch wurde in einem Perkin-Elmer Thermolcycler (PE 9600) folgendermaßen behandelt:

| | | |
|---|---|---|
| | 3 min | 94 °C |
| dann 40 Zyklen | 30 s | 94 °C |
| | 30 s | 50 °C |
| | 1 min | 72 °C |
| dann bei | | 4 °C. |

Die Amplifikate können bei 4 °C oder bei längerer Standzeit bei -20 °C aufbewahrt werden.

### D. Detektion

Die Amplifikate wurden mit dem Enzymun-Test DNA Detection (Boehriner Mannheim GmbH, Cat. No. 1447777) auf dem ES 300 (Boehringer Mannheim GmbH) nachgewiesen.

Dabei wurde ein Oligonukleotid, dessen Sequenz innerhalb des amplifizierten Bereiches liegt, und welches mit Hilfe von Biotin-Amidit link (Firma ABI, Best.-Nr. 401396) biotinyliert wurde, z. B. SEQ.ID.NO. 4 oder SEQ.ID.NO. 5, als Fangsonde verwendet. Die Hybride wurden an Streptavidin-Tubes (Boehringer Mannheim GmbH) gebunden und mit Anti-DIG-Antikörpern nachgewiesen. Hierzu wurde das PCR-Produkt im Verhaltnis 1 : 10 mit Denaturierungslösung verdünnt. Die Konzentration der Fangsonde betrug 75 ng/ml Hybridisierungspuffer. Ansonsten wurden die Vorschriften der Beipackzettel befolgt.

## Patentansprüche

1. HIV-1 spezifisches Oligonukleotid, das eine Sequenz enthält, die ausgewählt ist aus dem Bereich der Nukleotidpositionen 900 bis zum 3'-Ende des gag-Gens von HIV-1.

2. Oligonukleotid gemäß Anspruch 1, dadurch gekennzeichnet, daß die Sequenz ausgewählt ist aus dem Bereich der Nukleotidpositionen 900 bis zum 3'-Ende des gag-Gens von HIV-1, Subtyp B.

3. Oligonukleotid gemäß Anspruch 2, dadurch gekennzeichnet, daß die Sequenz zu mehr als 80 % homolog oder zu mehr als 80 % komplementär zu einer Basenfolge von mehr als 10 aufeinanderfolgenden Basen der SEQ.ID.NO. 1 ist.

4. Oligonukleotid gemäß einem der Ansprüche 1 bis 3, dadurch gekennzeichnet, daß es außerdem eine nicht HIV-spezifische Markierung enthält.

5. Set von Primern zur Amplifikation von HIV-1-Nukleinsäuren, dadurch gekennzeichnet, daß es mindestens 2 unterschiedliche Oligonukleotide gemäß einem der Ansprüche 1 bis 3 enthält.

6. Verfahren zur spezifischen Amplifikation von HIV-1-Nukleinsäuren mit Hilfe eines Sets von Primern, dadurch gekennzeichnet, daß Nukleinsäuren von mindestens 5 Subtypen von HIV-1 erkannt und amplifiziert werden.

7. Verfahren gemäß Anspruch 6, dadurch gekennzeichnet, daß als Primerset das Set gemäß Anspruch 5 eingesetzt wird.

8. Verfahren zum Nachweis von HIV-1 unter Amplifikation von HIV-1-Nukleinsäuresequenzen und Nachweis der Amplifikate, dadurch gekennzeichnet, daß die Amplifikation mit einem Primerset gemäß Anspruch 5 vorgenommen wird.

9. Verfahren gemäß Anspruch 8, dadurch gekennzeichnet, daß der Nachweis mit Hilfe einer Nukleinsäuresonde gemäß Anspruch 4 vorgenommen wird.


Sequenzprotokoll

(1) ALLGEMEINE ANGABEN:

(i) ANMELDER:
(A) NAME: Boehringer Mannheim GmbH
(B) STRASSE: Sandhofer Strasse 116
(C) ORT: Mannheim
(E) LAND: Germany
(F) POSTLEITZAHL: 68298
(G) TELEFON: 0621 759 4348
(H) TELEFAX: 0621 759 4457

(ii) BEZEICHNUNG DER ERFINDUNG: Primer und Probes zum Nachweis von
HIV

(iii) ANZAHL DER SEQUENZEN: 5
(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)
(2) ANGABEN ZU SEQ ID NO: 1:
(i) SEQUENZKENNZEICHEN:
(A) LÄNGE: 488 Basenpaare
(B) ART: Nucleotid
(C) STRANGFORM: Einzelstrang
(D) TOPOLOGIE: linear

(ii) ART DES MOLEKÜLS: Genom-RNA

(xi) SEQUENZBESCHREIBUNG: SEQ ID NO: 1:
CAAAAACCAA GAGCCGAGCA AGCCACAGGA GAAAAAAGGA GACAGAAACC CCGGCCAAAA 60
GCGAACCCAG AGAAAACAAA AAGCAGGGAC CAGCAGCACA CAGAAGAAAG AGACAGCAGC 120
AGGGAGAGGG GGACCCAGCC AAAAGCAAGA AGGCGAAGCA AGAGCCAAGA ACAAACAGCA 180
CCAAAGCGCA GAAAGGAAAG GGACCAAAGA AAAAGAAGGC AACGGGCAAA GAGGGCACAA 240
GCCAAAAAGC AGGGCCCCAG GAAAAAGGGC GGGAAAGGGA AAGGAAGGAC ACCAAAGAAA 300

GAGCACAAGA GGGACGACAG GCAAAGGGAA AACGGCCCCC ACAAGGGAAG GCCAGGGAAC 360
CCAGAGCAGA CCAGAGCCAA CAGCCCCACC AGAAGAGAGC CAGGGGGGAA GAGACAACCC 420
CCCAGAAGCA GGAGAAGAAG ACAAGGAACG ACCAGCCCCC AAACACCGGC AACGACCCAC 480
GCACAGAA 488

(2) ANGABEN ZU SEQ ID NO: 2:
(i) SEQUENZKENNZEICHEN:
(A) LÄNGE: 24 Basenpaare
(B) ART: Nucleotid
(C) STRANGFORM: Einzelstrang
(D) TOPOLOGIE: linear
(ii) ART DES MOLEKÜLS: Sonstige Nucleinsäure
(A) BESCHREIBUNG: /desc = "Oligodesoxyribonukleotid"

(xi) SEQUENZBESCHREIBUNG: SEQ ID NO: 2:
TGATGACAGC ATGTCAGGGA GTGG 24

(2) ANGABEN ZU SEQ ID NO: 3:
(i) SEQUENZKENNZEICHEN:
(A) LÄNGE: 24 Basenpaare
(B) ART: Nucleotid
(C) STRANGFORM: Einzelstrang
(D) TOPOLOGIE: linear

(ii) ART DES MOLEKÜLS: Sonstige Nucleinsäure
(A) BESCHREIBUNG: /desc = "Oligodesoxyribonukleotid"
(xi) SEQUENZBESCHREIBUNG: SEQ ID NO: 3:
TCCACATTTC CAACACCCCT TTTT 24

(2) ANGABEN ZU SEQ ID NO: 4:
(i) SEQUENZKENNZEICHEN:
(A) LÄNGE: 21 Basenpaare
(B) ART: Nucleotid
(C) STRANGFORM: Einzelstrang
(D) TOPOLOGIE: linear
(ii) ART DES MOLEKÜLS: Sonstige Nucleinsäure
(A) BESCHREIBUNG: /desc = "Oligodesoxyribonukleotid"

(xi) SEQUENZBESCHREIBUNG: SEQ ID NO: 4:
TTCAATTGTG GCAAAGAAGG G 21

(2) ANGABEN ZU SEQ ID NO: 5:
(i) SEQUENZKENNZEICHEN:
(A) LÄNGE: 22 Basenpaare
(B) ART: Nucleotid
(C) STRANGFORM: Einzelstrang
(D) TOPOLOGIE: linear
(ii) ART DES MOLEKÜLS: Sonstige Nucleinsäure
(A) BESCHREIBUNG: /desc = "Oligodesoxyribonukleotid"

(xi) SEQUENZBESCHREIBUNG: SEQ ID NO: 5:
AAAGCAAGAA TTTTGGCTGA AG 22